(19) Europäisches Patentamt
European Patent Office
Office européen des brevets

(11) Publication number: **0 051 681**
**B1**

(12) **EUROPEAN PATENT SPECIFICATION**

(45) Date of publication of patent specification: **31.07.85**

(21) Application number: **81901203.0**

(22) Date of filing: **01.05.81**

(86) International application number:
**PCT/JP81/00105**

(87) International publication number:
**WO 81/03331 26.11.81 Gazette 81/28**

(51) Int. Cl.$^4$: **C 07 D 493/04** //
(C07D493/04, 319:00)

# (54) PROCESS FOR PREPARING A DIBENZYLIDENESORBITOL OR A DIBENZYLIDENEXYLITOL.

(30) Priority: **16.05.80 JP 65601/80**

(43) Date of publication of application:
**19.05.82 Bulletin 82/20**

(45) Publication of the grant of the patent:
**31.07.85 Bulletin 85/31**

(84) Designated Contracting States:
**FR**

(56) References cited:
**US-A-3 721 682**

**Chemical Abstracts. Vol. 47, No. 7, (1953-4-10), (Columbus, Ohio, U.S.A.), H. Hagiwara "Chemical Structure of Dibenzalsorbitol" 3235g, Journal of the Pharmaceutical Society of Japan Vol. 72, No. 7 p. 929-30 (1952)**

(73) Proprietor: **NEW JAPAN CHEMICAL CO.,LTD.**
**13, Yoshijima Yaguracho Fushimi-ku**
**Kyoto-shi Kyoto 612 (JP)**

(72) Inventor: **KOBAYASHI, Toshiaki**
**1-21, Hirata Tanabecho Tsuzuki-gun**
**Kyoto 610-03 (JP)**
Inventor: **FUJITANI, Kango**
**6-128, Hiramori Okubocho, Uji-shi**
**Kyoto 611 (JP)**
Inventor: **MURAI, Koichi**
**6-6, Tenjin 4-chome Nagaokakyo-shi**
**Kyoto 617 (JP)**

(74) Representative: **Ores, Irène et al**
**CABINET ORES 6, Avenue de Messine**
**F-75008 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 051 681 B1

**Description**

Technical Field

The present invention relates to a process for preparing dibenzylidene sorbitols and dibenzylidene xylitols, and more particularly to a process for preparing dibenzylidine sorbitol, dibenzylidine xylitol and their nuclear substitution compounds.

Background Art

Dibenzylidene sorbitol, dibenzylidene xylitol and nuclear substitution compounds thereof have unique properties as additives and are used, for example, as transparency imparting agents for polypropylene resins, anti-rutting agents for asphalt pavements, precipitation preventing agents for preparing and storing coal-and-oil mixture fuels, flowability improving agents for PVC paste sols, flowability improving agents for FRP, excipients for pharmaceuticals, cosmetics, marking agents, fuel solidifying agents, etc. Among the benzylidene sorbitol, benzylidene xylitol and nuclear substitution compounds thereof, especially the 1,3- and 2,4-substituted compounds represented by the following structural formula have the properties useful as such additives.

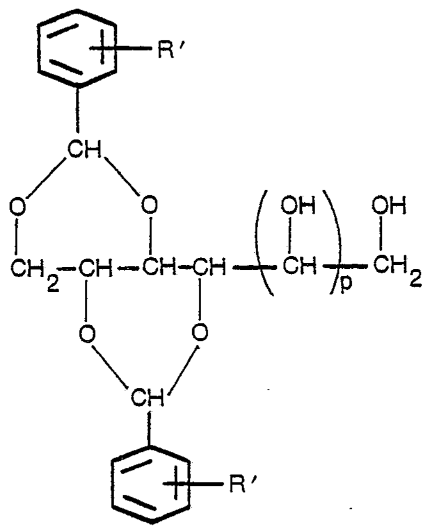

wherein R′ is an alkyl group, alkoxy group, halogen atom or nitro group, and p is an integer of 1 or 0.

Accordingly it is important to prepare such 1,3- and 2,4-substituted compounds in high yields and with high selectivities.

Dibenzylidine sorbitol is usualy produced by the dehydration condensation reaction of sorbitol and benzaldehyde. Processes have been developed in recent years for carrying out this reaction in the form of a slurry with use of cyclohexane and/or a saturated hydrocarbon having 6 to 10 carbon atoms in a large amount as the reaction medium (Published Examined Japanese Patent Applications No. 43748/1973 and No. 14758/1974).

It is also known by U.S. Patent 3 721 682, to produce benzylidene sorbitol by means of a process which comprises reacting an aqueous solution of sorbitol with benzaldehyde in the presence of a dehydrating acid catalyst, while adding an hydrophobic solvent such as cyclohexane, an organic water-soluble polar solvent such as inter alia methanol, whereby an azeotropic mixture of cyclohexane and water is formed by heating under stiring the reaction system, said azeotropic mixture being condensed and separated to remove the water from the reaction system and recycle the cyclohexane to the system, whereas the resultant benzylidene sorbitol thus produced is recovered; according to this process, cyclohexane is added to the reaction system in an amount of 5 to 20 parts by weight based on 1 part by weight of the benzaldehyde and the polar solvant is issued in an amount of 1,0 to 10% by weight, based on the weight of the cyclohexane. These processes utilize the properties of the reaction medium that it is azeotropic with water and free of gelation by the resulting benzilidene sorbitol. More specifically when the reaction medium of cyclohexane and/or saturated hydrocarbon with 6 to 10 carbon atoms is used, the water resulting from the reaction is continuously withdrawn from the system by an azeotropic phenomenon to promote the dehydration reaction, while the product separates out and disperses in the reaction medium in the form of pearl-like particles without gelling or solidifying the medium, permitting the reaction to proceed in the state of a slurry having a low viscosity. Accordingly the reaction can be carried out efficiently in a reactor equipped with a usual impeller mixer while assuring good dispersion of the charge, consequently giving the desired product in a yield of at least about 70% within a relatively short period of time, i.e. 5 to 7 hours. For the reaction medium to be serviceable as a dispersing agent in the processes, the medium is limited to cyclohexane and/or saturated hydrocarbons having 6 to 10 carbon atoms which will not be gelled

or solidified by the resulting benzylidene sorbitol. Furthermore it is critical that the medium be used in a large amount to maintain the reaction system in the form of a slurry as mentioned above.

Our research has revealed the totally unexpected fact that although the reaction system becomes a gel to solid phase when cyclohexane and/or saturated hydrocarbon are/is used in a small amount, the reaction system, if forcibly agitated, affords in a high yield and with a high selectivity the desired compound, especially 1,3- and 2,4-substituted compounds having the above-mentioned unique properties as additives. More specifically when a system comprising sorbitol or xylitol, a benzaldehyde or an alkyl acetal derivative thereof, a catalyst and a water-soluble polar solvent according to the conventional process is subjected to reaction with addition of a small amount of the hydrophobic organic solvent, the resulting dibenzylidene sorbitols or dibenzylidene xylitols separate out as crystals while gelling or solidifying the reaction system to render the hydrophobic organic solvent no longer serviceable as the dispersing medium as used in the conventional process. Surprisingly, however, when the reaction system in the form of a gel to solid phase is forcibly agitated, the system gives in high yields and with high selectivities dibenzylidene sorbitols or dibenzylidene xylitols which have the aforementioned unique properties as additives. Our research has further revealed that such a result is achieved not only when using cyclohexane, saturated hydrocarbons, etc. which will not be gelled by dibenzylidene sorbitols or dibenzylidene xylitols but also when using hydrophobic solvent alike which will inherently be gelled or solidified by such compounds. The present invention has been accomplished based on such novel findings.

Disclosure of the Invention

The present invention relates to a process for preparing dibenzylidene sorbitols or dibenzylidene xylitols by reacting sorbitol or xylitol with a substituted or unsubstituted benzaldehyde or alkyl acetal derivative thereof in the presence of an acid catalyst, a hydrophobic organic solvent and a water-soluble organic polar solvent, the process being characterized by using the hydrophobic organic solvent in an amount sufficient to permit the resulting dibenzylidene sorbitols or dibenzylidene xylitols to separate out as crystals and capable of maintaining the reaction system in the form of a gel or semi-solid to solid phase, by conducting the reaction with forced agitation in a kneader at a temperature of 30 to 180°C whereby the amount of the hydrophobic organic solvent is 40 to 200 parts by weight per 100 parts by weight of the starting material and the amount of the water soluble organic polar solvent is 5 to 300 parts by weight of the starting material.

The reaction of this invention, although effected in the state of a gel or semi-solid to powdery or to solid phase, gives the desired product in a high yield of at least about 80% and with a high purity of at least about 90% at all times. Moreover the reaction can be carried out within as short a period as 3 hours, whereas the foregoing conventional processes required 5 to 7 hours. This is very inconceivable in chemistry in which it is generally thought that the reaction in a gel to solid phase usually involves reduced dispersion and reduced reactivity of the starting material, resulting in lower yield and selectivity and necessitating a prolonged period of time. The invention has other advantages in that the process can be practiced with use of a compact reactor and ensures savings in energy.

According to the invention, sorbitol or xylitol is usable in any of various forms, i.e. in the form of a dilute or concentrated aqueous solution or a solid in which it is usually available commercially.

The benzaldehydes to be reacted with sorbitol or xylitol are not particularly limited and are a wide variety of those presently used for producing the dibenzylidene sorbitols or dibenzylidene xylitols having the aforementioned unique properties as additives.

Preferred examples are substituted or unsubstituted benzaldehydes represented by the following formula

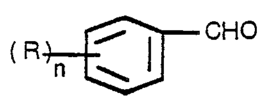

wherein R is a hydrogen atom, lower alkyl group, lower alkoxy group, halogen atom or nitro group, and n is an integer of 1 to 3.

In addition to benzaldehydes, alkyl acetal derivatives thereof are also usable in this invention. Such alkyl acetal derivatives can be prepared by a usual process, for example, by reacting benzaldehydes with a lower alcohol having 1 to 5 carbon atoms or a lower alkyl ester of o-formic acid. The benzaldehyde or alkyl acetal derivative thereof is used in an amount of 1 to 2.8 moles, preferably 1.5 to 2.5 moles, per mole of sorbitol or xylitol.

Acid catalysts usable for this invention are any of those usually used for producing benzylidene sorbitols. Typical of such acid catalysts are, for example, sulfuric acid, p-toluenesulfonic acid, phosphoric acid, hydrochloric acid, zinc chloride, $C_{2-12}$ alkyl benzene-sulfonic acids, G-acid, L-acid, etc. The acid catalyst is used usually in an amouint of 0.05 to 10 parts by weight, preferably 0.2 to 3 parts by weight, per 100 parts by weight of sorbitol or xylitol, and a benzaldehyde or alkyl acetal derivative thereof (hereinafter referred to simply as "materials").

According to the invention, hydrophobic organic solvents serve to cause the dibenzylidene sorbitols or dibenzylidene xylitols which are formed with the progress of the reaction to separate out as crystals. The

solvents also perform the function of maintaining the reaction system in the form of a gel to solid phase but do not serve the function of the dispersing medium used in the conventional processes. Preferably such hydrophobic organic solvents are azeotropic with water from the viewpoint of removal of water from the reaction system. Useful hydrophobic organic solvents are not limited to those which will not be gelled or solidified by the product; solvents which will be gelled or solidified by the products are also usable if they dissolve the benzaldehydes used as materials, are hydrophobic but will not be easily decomposed by the acid catalyst. Examples of useful solvents which are free of gelation or solidification by the product are cyclohexane, cyclohexanes substituted with lower alkyl such as methylcyclohexane, ethylcyclohexane, etc., chain saturated hydrocarbons having 6 to 16 carbon atoms such as n-hexane, kerosene, heptane, octane, decane, dodecane, etc., carbon tetrahalides such as carbon tetrachloride, etc. These solvents are used singly, or at least two of them are usable in admixture. Examples of useful solvents which will be gelled or solidified by the product are aromatic hydrocarbons such as benzene, toluene, xylene, ethylbenzene, mesitylene, cumene, pseudocumene, diphenyl, etc.; hydrocarbon halides such as chloroform, 1,2-dichloroethane, 1,2-dichloropropane, 1-chloropropane, 1-chlorobutane, 1-chloro-1-methylpropane, trichloroethylene, dichloroethylene, chlorobenzene, etc.; ethers such as isopropyl ether, isoamyl ether, methyl butyl ether, 1,2-dimethoxyethane, 1,2-diethoxyethane, anisole, phenetole, diphenyl ether, etc.; nitro compounds such as nitromethane, nitrobenzene, etc.; esters such as methyl benzoate, butyl benzoate, etc.; ketones such as di-t-butyl ketone; decalin; etc. These can be used singly, or at least two of them are usable in admixture. These hydrophobic organic solvents are used in small amounts, but too small an amount of such a solvent, if used, will not permit the desired product to separate out as crystals, turning the reaction system to a syrup-like consistent liquid and failing to afford the desired product in a high yield and with a high selectivity. Accordingly the hydrophobic organic solvent is used in an amount sufficient to cause the product to separate out as crystals. This amount is usually at least 40 parts by weight, preferably at least 45 parts by weight, per 100 parts by weight of the materials, although variable in accordance with the kinds of the materials, the kind and amount of the water-soluble organic polar solvent, the reaction conditions, etc. From the viewpoint of reaction efficiency, the hydrophobic organic solvent is used in such an amount as to maintain the reaction system in the form of a gel to solid phase. Although this amount is also variable according to the kinds of the materials used, the reaction conditions, the kind and amount of the water-soluble organic polar solvent, etc., the amount is generally up to 200 parts by weight, preferably up to 150 parts by weight, per 100 parts by weight of the materials, whereby the reaction can be conducted in a satisfactory gel to solid phase.

It is thought that the water-soluble organic polar solvent used in this invention provides the site of reaction by dissolving the materials, namely sorbitol or xylitol and benzaldehydes. In fact, the reaction almost fails to proceed in the absence of this solvent. Preferred water-soluble organic polar solvents are protic solvents including alcohols such as methanol, ethanol, propanol, isopropanol, butanol, t-butanol, n-amyl alcohol, isoamyl alcohol, etc.; and glycol ethers such as ethylene glycol monomethyl ether, monoethyl ether, monopropyl ether and monobutyl ether, etc. Also usable are aprotic solvents such as N,N-dimethylformamide, N,N-dimethylacetamide, N-methylpyrrolidone, sulfolane, dimethylsulfolane, dioxane, cyclohexanone or diglyme. The protic solvent and the aprotic solvent can be used in combination. Such a water-soluble organic polar solvent is used usually in an amount of 5 to 300 parts by weight per 100 parts by weight of the materials, although the amount is variable in accordance with the kinds of the materials used, the reaction conditions, etc. In the case of the protic solvent, the amount is preferably 50 to 300 parts by weight, more preferably 80 to 200 parts by weight, per 100 parts by weight of the materials. In the case of the aprotic solvent, the amount is preferably 5 to 50 parts by weight, more preferably 7 to 40 parts by weight, per 100 parts by weight of the material.

Generally the present invention is practiced in the following manner. Since the reaction of the process of the invention is conducted in a gel to solid phase, usual impeller mixers are not usable, and there is the need to use a reactor equipped with a forced agitator, such as a kneader, a versatile mixer having a kneading device. Also usable are rotary press drier for slurry, etc. The reactor is preferably one having a gas inlet and a condenser equipped with a decanter. First, the materials (sorbitol or xylitol, and a benzaldehyde or alkyl acetal derivative thereof), an acid catalyst, a hydrophobic organic solvent and a water-soluble organic polar solvent are charged into the reactor. These may be charged in any order desired. Preferably the air in the reactor is replaced by an inert gas, such as nitrogen gas.

The mixture placed in is then agitated for reaction. The reaction temperature is generally 30 to 180°C, preferably 50 to 150°C, although variable in accordance with the kinds and amounts of the hydrophobic organic solvent and the water-soluble organic polar solvent, etc. With the progress of the reaction, the water resulting from the condensation reaction forms an azeotropic mixture with the hydrophobic organic solvent and is distilled off from the system, or the water is distilled off according to vapor-liquid equilibrium. When an alkyl acetal derivative of a benzaldehyde is used as one of the materials, the alcohol released by acetal exchange reaction also forms an azeotropic mixture with water and/or the hydrophobic organic solvent and is thereby removed from the system, or is distilled off according to vapor-liquid equilibrium. The water-soluble polar solvent also is partly removed similarly. These fractions are condensed by the condenser, and the condensate is separated into a hydrophobic organic solvent layer and an aqueous layer. The solvent layer can be recycled through the reaction system. The aqueous layer is withdrawn from the reactor. As the reaction proceeds, the resulting product separates out as crystals, while the hydrophobic organic solvent and water-soluble organic polar solvent in the reactor become gelled or solidified. The hydrophobic organic solvent, when free of gelation or solidification by the product, is absorbed or adsorbed by the product. Thus in the initial or intermediate stage of the reaction, the reaction system becomes a gel to solid phase, which, when forcibly agitated continuously, permits the reaction to proceed further. Generally the reaction is completed within a short period of time, i.e. 3 hours.

The reaction mixture obtained in the form of a gel to solid phase is treated by a usual method, for example, with an aqueous alkali hydroxide solution to neutralize the acid catalyst, then washed with hot water and/or an aqueous solution of surfactant to remove the unreacted materials, intermediate and organic polar solvent, and thereafter filtered. The desired product is obtained after drying.

The present invention will be described in greater detail with reference to the following examples and comparison examples, in which the purity and composition of the reaction products are determined by chromatography.

Examples 1—7

Sorbitol and benzaldehyde in a combined amount of 100 parts by weight and in the mole ratio listed in Table 1 are placed into a kneader having a thermometer, nitrogen inlet and condenser having a decanter. Subsequently cyclohexane and methanol in the amounts listed in Table 1 and 0.5 part by weight of 98% sulfuric acid serving as a catalyst are placed into the kneader. The materials are reacted at 78 to 82°C in nitrogen atmosphere. The water formed is distilled off as an azeotropic mixture along with cyclohexane and methanol. The cyclohexane condensed and separated off by the condenser is recycled through the reaction system, while the aqueous layer is withdrawn from the system. In this way, the materials are reacted for 3 hours, giving a reaction mixture which appears semisolid to powdery. The composition of the reaction mixture is analyzed with the result shown in Table 1. The reaction mixture is neutralized with an aqueous KOH solution, washed with hot water and filtered to obtain a product upon drying. Table 1 also shows the yield of the product.

Comparison Examples 1—3

The same reaction procedure as in Examples 1—7 is repeated with the exception of using sorbitol, benzaldehyde, cyclohexane and methanol in the charge mole ratios or amounts listed in Table 1. Table 1 shows the appearance and composition of each reaction mixture obtained by the three-hour reaction, and the yield of the product obtained by treating the mixture in the same manner as above.

TABLE 1

| | Cyclo-hexane (wt. parts) | Sorbitol/ benzaldehyde (charge mol ratio) | Methanol (wt. parts) | Appearance of reaction mixture | Composition of reaction mixture (wt. %) | | | | Yield (%) |
|---|---|---|---|---|---|---|---|---|---|
| | | | | | Sorbitol | 1) MBS | 2) DBS | 3) Isomers | |
| Comp. Ex. 1 | 0 | 1.0/2.0 | 150 | Syrup | 7.5 | 19.2 | 52.5 | 20.8 | 61 |
| ,, 2 | 25 | 1.0/2.0 | 120 | ,, | 3.2 | 12.6 | 72.5 | 11.7 | 80 |
| Example 1 | 43 | 1.2/2.0 | 120 | Powder | 0.3 | 2.3 | 94.9 | 2.5 | 95 |
| ,, 2 | 55 | 1.0/2.5 | 180 | ,, | 0.3 | 2.0 | 97.5 | 0.2 | 96 |
| ,, 3 | 70 | 1.0/1.8 | 150 | ,, | 0.3 | 1.5 | 98.0 | 0.3 | 97 |
| ,, 4 | 100 | 1.0/2.0 | 100 | ,, | 0.4 | 1.8 | 97.5 | 0.3 | 97 |
| 5 | 120 | 1.0/2.0 | 300 | Semisolid | 1.0 | 2.8 | 96.0 | 0.2 | 94 |
| 6 | 150 | 1.0/2.0 | 60 | ,, | 2.5 | 6.2 | 91.0 | 0.3 | 90 |
| 7 | 200 | 1.0/2.0 | 200 | ,, | 4.0 | 5.0 | 92.5 | 0.5 | 80 |
| Comp. Ex. 3 | 250 | 1.0/2.0 | 120 | Slurry | 7.2 | 9.0 | 83.2 | 0.6 | 75 |

1) MBS: monobenzylidene sorbitol
2) DBS: dibenzylidene sorbitol (1,3- and 2,4-compounds)
3) Isomers: extract obtained by Soxhlet extraction method with use of cyclohexane

Table 1 reveals that the reaction, when conducted in a gel to solid phase, gives the desired product in a high yield with a high purity. The result of Comparison Example 1 indicates that the use of the hydrophobic organic solvent is critical.

Comparison Example 4

The same reaction procedure as in Example 1 is repeated with the exception of using xylitol (1 mole) and benzaldehyde dimethyl acetal (2 moles) in a combined amount of 100 parts by weight, 100 parts by weight of methanol and 1.0 part by weight of 98% concentrated sulfuric acid. The reaction is conducted for 6 hours, giving a reaction mixture which appears to be a consistent uniform liquid. When neutralized, washed with hot water, filtered and dried, the mixture affords a solid product in a yield as low as 52%. The product contains about 20% of isomers soluble in cyclohexane.

Example 8

Sorbitol (28.8 g), 31.5 g of benzaldehyde, 0.5 g of 50% sulfuric acid, 5 ml of N,N-dimethylformamide and 70 ml of cyclohexane are placed into the kneader used in Example 1. The materials are reacted in nitrogen atmosphere at 82°C with forced agitation. The cyclohexane is distilled off as an azeotropic mixture with water, then condensed and separated off by the condenser, and thereafter recycled through the reaction system. The reaction system becomes a semisolid phase with the progress of the reaction and appears powdery in 3 hours. The reaction mixture is cooled, neutralized with an aqueous KOH solution, washed with hot water and filtered. The product, when dried, gives 50 g of 1,3- and 2,4-dibenzylidene sorbitols. Yield: 95%. Purity: 99%.

Comparison Example 5

The same reaction procedure as in Example 8 is repeated without using any N,N-dimethylformamide. However, the reaction system becomes neither gelled nor semisolidified but separates into two layers, failing to afford the desired dibenzylidene sorbitol since hardly any reaction occurs.

Example 9

The same reaction procedure as in Example 8 is repeated except that benzaldehyde is replaced by 60 g of 2,4,5-trimethylbenzaldehyde dimethyl acetal. The methanol formed is distilled off. The materials are reacted for 3 hours, affording a powdery reaction mixture, which is similarly after-treated, whereby di-(trimethylbenzylidene)sorbitol is obtained in a yield of 93% with a purity of 98%.

Example 10

A semisolid reaction mixture is obtained in the same manner as in Example 8 with the exception of using 26.8 g of sorbitol, 35.0 g of p-tolualdehyde, 0.4 g of p-toluenesulfonic acid, 85 ml of n-heptane and 200 ml of n-butanol and reacting the materials at 95°C. The mixture is similarly after-treated to give di-(p-methylbenzylidene)sorbitol in a yield of 96% with a purity of 97%.

Example 11

The reaction procedure of Example 8 is repeated in the same manner with the exception of using 26.8 g of sorbitol, 48.0 g of p-isopropylbenzaldehyde, 1.0 g of $C_9$ alkylbenzenesulfonic acid, 9 ml of dimethylsulfoxide, 120 ml of benzene and 100 ml of isopropanol. The reaction is conducted in a semisolid phase for 3 hours, giving di-(p-isopropylbenzylidene)sorbitol in a yield of 93% with a purity of 95%.

Example 12

A powdery reaction mixture is obtained in the same manner as in Example 8 with the exception of using 24.0 g of xylitol, 41.5 g of p-chlorobenzaldehyde, 0.5 g of 50% sulfuric acid, 60 ml of trichloroethylene and 18 ml of dioxane. The mixture is similarly after-treated, affording di-(p-chlorobenzylidene)xylitol in a yield of 92% with a purity of 97%.

Example 13

The same procedure as in Example 8 is repeated with the exception of using 26.8 g of sorbitol, 39.5 g of 2,4-dimethylbenzaldehyde, 50 ml of cyclohexane, 0.4 g of p-toluenesulfonic acid, 30 ml of anisole and 100 ml of ethanol. The reaction is conducted for 2.5 hours, giving a powdery reaction mixture, which is similarly after-treated to obtain di-(2,4-dimethylbenzylidene)sorbitol in a yield of 93% with a purity of 98%.

Example 14

A semisolid reaction mixture is obtained in the same manner as in Example 8 with the exception of using 26.8 g of sorbitol, 39.3 g of p-ethylbenzaldehyde, 1.2 g of dodecylbenzenesulfonic acid, 5.0 ml of N,N-dimethylformamide and 80 ml of nitrobenzene. The mixture is thereafter similarly treated to give di-(p-ethylbenzylidene)sorbitol in a yield of 96% with a purity of 95%. The reaction time is 2.5 hours.

Example 15

The same procedure as in Example 8 is repeated with the exception of using 30.3 g of sorbitol, 45.7 g of p-methoxybenzaldehyde, 1.0 g of p-toluenesulfonic acid, 8 ml of cyclohexanone, 175 ml of $C_8$—$C_{10}$ saturated hydrocarbons and 8 ml of amyl alcohol. The materials are reacted for 3 hours, giving a semisolid reaction mixture. The mixture is similarly treated thereafter, affording di-(p-methoxybenzylidene)sorbitol in a yield of 92% with a purity of 99%.

Example 16

The same procedure as in Example 8 is repeated with the exception of using 30.3 g of sorbitol, 44.0 g of p-nitrobenzaldehyde, 1.5 g of p-toluenesulfonic acid, 5 ml of N-methylpyrrolidone, 150 ml of methylcyclohexane and 20 ml of ethylene glycol monomethyl ether. The materials are reacted for 2.7 hours, giving a semisolid paste. The paste is thereafter treated similarly to afford di-(p-nitrobenzylidene)sorbitol in a yield of 91% with a purity of 97%.

**Claims**

1. A process for preparing dibenzylidene sorbitols or dibenzylidene xylitols by reacting sorbitol or xylitol with a substituted or unsubstituted benzaldehyde or alkyl acetal derivative thereof in the presence of an acid catalyst, a hydrophobic organic solvent and a water-soluble organic polar solvent, the process being characterized in that the reaction is conducted in a kneader with forced agitation at a temperature of 30 to 180°C by using the hydrophobic organic solvent in an amount of 40 to 200 parts by weight per 100 parts by weight of the starting material and the water-soluble organic polar solvent in an amount of 5 to 300 parts by weight per 100 parts by weight of the starting material to permit the resulting dibenzylidene sorbitols or dibenzylidene xylitols to separate out as crystals and to maintain the reaction system in the form of a semi-solid phase.

2. A process as defined in claim 1 wherein the hydrophobic organic solvent is at least one of cyclohexanes having or not having a lower alkyl substituent, chain saturated hydrocarbons having 6 to 16 carbon atoms and carbon tetrahalides.

**Patentansprüche**

1. Verfahren zur Herstellung eines Dibenzylidensorbitols oder eines Dibenzylidenxylitols durch Umsetzung von Sorbitol oder Xylitol mit einem substituierten oder unsubstituierten Benzaldehyd oder Alkylacetalderivat davon in Gegenwart eines sauren Katalysators, eines hydrophoben organischen Lösungsmittels und eines wasserlöslichen organischen polaren Lösungsmittels, dadurch gekennzeichnet, daß man die Reaktion in einem Kneter mit Zwangsdurchbewegung bei einer Temperatur von 30 bis 180°C unter Verwendung des hydrophoben organischen Lösungsmittles in einer Menge von 40 bis 200 Gewichtsteilen pro 100 Gewichtsteile Ausgangsmaterial und unter Verwendung des wasserlöslichen organischen polaren Lösungsmittels in einer Menge von 5 bis 300 Gewichtsteilen pro 100 Gewichtsteile Ausgangsmaterial durchführt, um die resultierenden Dibenzylidensorbitole oder Dibenzylidenxylitole als Kristalle abscheiden zu lassen und um das Reaktionssystem in Form einer halbfesten bis festen Phase aufrechtzuerhalten.

2. Verfahren nach Anspruch 1, dadurch gekennzeichnet, daß das hydrophobe organische Lösungsmittel mindestens ein Cyclohexan mit oder ohne einen Niedrigalkylsubstituenten, ein kettengesättigter Kohlenwasserstoff mit 6 bis 16 Kohlenstoffatomen und ein Kohlenstofftetrahalogenid ist.

**Revendications**

1. Procédé de préparation de dibenzylidène sorbitols ou de dibenzylidène xylitols par réaction de sorbitol ou de xylitol avec un benzaldéhyde substitué ou non-substitué ou un dérivé alcoyl-acétal de celui-ci, en présence d'un catalyseur acide, d'un solvant organique hydrophobe et d'un solvant polaire organique soluble dans l'eau, caractérisé en ce que la réaction est effectuée dans un malaxeur avec une agitation forcée, à une température de 30 à 180°C, avec une quantité de 40 à 200 parties en poids du solvant organique hydrophobe pour 100 parties en poids de la matière de départ et une quantité de 5 à 300 parties en poids du solvant polaire organique soluble dans l'eau pour 100 parties en poids de la matière de départ, pour que les dibenzylidène sorbitols ou les dibenzydilène xylitols résultants puissent se séparer sous forme de cristaux et pour que le système de réaction soit maintenu sous la forme d'une phase semi-solide à solide.

2. Procédé suivant la Revendication 1, caractérisé en ce que le solvant organique hydrophobe est au moins un solvant choisi parmi des cyclohexanes portant ou non un substituant alcoyle inférieur, des hydrocarbures satinés, contenant 6 à 16 atomes de carbone et des tétrahalogénures de carbone.